# EUROPEAN PATENT APPLICATION

(11) **EP 4 497 424 A1**
(43) Date of publication of application: **29.01.2025**
(21) Application number: 24190089.3
(22) Date of filing: 22.07.2024
(51) Int. Cl.: A61F 13/15

(54) **APPARATUS AND METHOD FOR PRODUCING AN ABSORBENT SANITARY ARTICLE**

(30) Priority: 27.07.2023 IT 202300015849
(71) Applicant: GDM S.p.A., 40133 Bologna (IT)
(72) Inventor: ZAVALLONI, Alessandro, I-40133 Bologna (IT); TOSCANI, Federico, I-40133 Bologna (IT); ROSSI, Eros, I-40133 Bologna (IT)
(74) Representative: Porta & Consulenti Associati S.p.A.

(57) **Abstract**

The invention concerns an apparatus (100) for producing an absorbent sanitary article, comprising a slick support surface (202), a forming unit (190) configured to form a multilayer web (30) of an absorbent sanitary article, the multilayer web (30) comprising a top sheet web (112), a back sheet web (114) and a plurality of absorbent cores placed between said top sheet web (112) and said back sheet web (114), and to feed said multilayer web (30) on said support surface (202), at least one glue dispenser (194, 196) configured to dispense a liquid glue onto said back sheet web (114) and/or onto said top sheet web (112). The apparatus (100) further comprises at least one compression roller (210; 220) adjacent to said support surface (202) and configured to compress said multilayer web (30) on said support surface (202), said at least one compression roller (210; 220) comprising a slick cylindrical outer surface (212; 222).

## Description

The present invention refers to an apparatus and method for producing an absorbent sanitary article.

Absorbent sanitary articles are for example female sanitary napkins, baby diapers, diapers and pads for people suffering from incontinence and the like. In the following description, reference will be made to the non-limiting example of female sanitary napkins.

Structurally, a female sanitary napkin comprises a plurality of elements superimposed on each other.

The female sanitary napkin is applied, through its first adhesive portions, inside an undergarment worn by the user, by folding and turning down two opposite flaps on the outside of the garment and by fixing them to it with respective second adhesive portions.

Typically, the female sanitary napkin comprises a top sheet and a back sheet superimposed and joined together. An absorbent core is arranged between the top sheet and the back sheet.

The top sheet is made of a material permeable to body fluids and is configured to be arranged facing the wearer's body, in contact with it, when the sanitary towel is applied to the undergarment.

The absorbent core has the function of absorbing the body fluids that pass through the top sheet and retaining them with no leaks even for several hours. The absorbent core comprises an absorbent material typically in granular or powder form, called SAP (acronym for "Super Absorbent Powder"), capable of absorbing liquids up to 300 times its own weight and up to 60 times its own volume.

The back sheet is configured to be applied to the undergarment via the first adhesive portions and is made impermeable to body fluids to avoid the leak of the liquids absorbed by the absorbent core.

For producing an absorbent article, a multilayer web is formed by superimposing together a back sheet web, in the form of a continuous web, and a top sheet web, in the form of a continuous web, with a plurality of absorbent cores in succession placed between them. The top sheet and back sheet are joined together and subsequently the multilayer web is cut around each absorbent core in order to produce a plurality of semi-finished absorbent article products. For each semi-finished absorbent article product, the cut top sheet web defines the top sheet of the absorbent article, the cut back sheet web defines the back sheet of the absorbent article, and the absorbent core remains placed therebetween.

Conventionally, the top sheet and back sheet webs of the multilayer web are joined by heat-sealing carried out by compressing the top sheet and back sheet webs together, at the edges of the absorbent core, by means of knurled or dotted rollers, optionally with the addition of conventional synthetic-based glues. Conventional synthetic-based glues at room temperature are solid and are temporarily heated to pass to the liquid state for application.

The Applicant has observed that conventional glues once applied penetrate and diffuse relatively poorly within the multilayer web as they have relatively high viscosity and solidify relatively quickly.

In markets that are more sensitive to environmental issues, regulators and consumers prefer biodegradable products.

Therefore, biodegradable female sanitary napkins of the biodegradable type have recently been conceived that can be disposed of in toilet bowls and dissolved in water in a time of the order of magnitude of some days, weeks or months. In these products, the two top sheet and back sheet webs and the absorbent core are made of biodegradable materials, for example cellulose-based.

The Applicant has found that the webs made of biodegradable materials are not heat-sealable and that the conventional synthetic-based glues are not biodegradable.

The Applicant has realised that, in order to join the top sheet and back sheet webs and the absorbent core together while maintaining the biodegradable properties of the product, biodegradable type glues can be used instead of conventional synthetic-based glues.

The Applicant has found that the biodegradable glues currently in use are typically water-based and, at room temperature, are liquid and have a particularly low viscosity, unlike conventional glues. The biodegradable glues also dry out in generally longer times, even on the order of a few days, and therefore remain liquid significantly longer than the conventional glues after application. The biodegradable glues therefore have a generally lower adhesion power than conventional glues during the post-application processing steps and at least until they harden.

The Applicant has found that, in order to adequately make adhere the top sheet and back sheet webs in biodegradable material by means of biodegradable glues, it is necessary to use a greater amount of glue and to press the top sheet and back sheet webs together after the application of the glue.

However, the Applicant has verified that, by compressing the multilayer web in a conventional manner after the application of the biodegradable type glue, the glue tends to penetrate through the top sheet and back sheet webs and to impregnate the absorbent core, resulting in greater stiffness of the product that may cause discomfort to the user.

The present invention therefore concerns, in a first aspect thereof, an apparatus for producing an absorbent sanitary article.

Preferably, a slick support surface is provided.

Preferably, a forming unit configured to form a multilayer web of an absorbent sanitary article comprising a top sheet web, a back sheet web and a plurality of absorbent cores placed between said top sheet web and said back sheet web is provided.

Preferably, the forming unit is configured to feed said multilayer web on said support surface.

Preferably, at least one glue dispenser configured to dispense a liquid glue onto said back sheet web and/or onto said top sheet web is provided.

Preferably, at least one compression roller adj acent to said support surface and configured to compress said multilayer web on said support surface is provided.

Preferably, said at least one compression roller comprises a slick cylindrical outer surface.

The compression roller with the slick cylindrical outer surface compresses the multilayer web on the slick support surface. The Applicant has found that the absence of asperities on the outer surface of the compression roller results in a uniform compression that reduces the penetration of the glue through the top sheet and back sheet webs of the multilayer web and into the absorbent core and avoids areas with undesired concentrations of glue.

In a second aspect thereof, the present invention concerns a method for producing an absorbent sanitary article.

Preferably, a multilayer web of an absorbent sanitary article comprising a top sheet web, a back sheet web, and a plurality of absorbent cores placed between said top sheet web and said back sheet web is formed.

Preferably, said multilayer web is fed on a slick support surface.

Preferably, a biodegradable liquid glue is dispensed onto said back sheet web and/or onto said top sheet web.

Preferably, said multilayer web is compressed between at least one compression roller and said support surface.

Preferably, said at least one compression roller comprises a slick cylindrical outer surface.

Preferably, the method in accordance with the second aspect of the present invention may be implemented by employing an apparatus in accordance with the first aspect of the present invention.

In the following of the present description and in the following claims, by "slick surface" (in particular, slick support surface, slick cylindrical outer surface and slick raised surface) is meant a uniform surface free of recesses, protrusions, knurls, holes, asperities and other obvious non-uniformities, and with a surface roughness of less than 100 µm, preferably less than 10 µm.

In the following of this description and in the following claims, expressions like "upstream", "downstream" and similar refer to the movement path within the apparatus of the multilayer web and of the individual elements that compose it, unless otherwise specified.

The present invention may have, in both aspects discussed above, at least one of the preferred features described below. Such features may thus be present individually or in combination with each other, unless expressly stated otherwise, both in the apparatus of the first aspect of the present invention and in the method of the second aspect of the present invention.

Preferably, said slick cylindrical outer surface defines the entire lateral surface of said at least one compression roller.

Preferably, a first glue dispenser configured to dispense a liquid glue onto said top sheet web is provided.

Preferably, a second glue dispenser configured to dispense a liquid glue onto said back sheet web is provided.

In this way, a better bonding between top sheet web and back sheet web is obtained.

Preferably, the back sheet web and/or the top sheet web is impregnated with liquid glue prior to forming the multilayer web.

Preferably, said at least one compression roller is mounted at a respective adjustable distance from said support surface.

The Applicant has verified that the smaller the distance between the compression roller and the support surface, the greater the compression to which the multilayer web is subjected and the better the adherence obtained between the layers of the multilayer web. The Applicant has found that excessively compressing the multilayer web could promote glue leakages in the absorbent core. By allowing adjustment of the distance between the compression roller and the support surface, it is possible to adjust a compression level balancing the needs of reducing leakages and improving adherence.

Preferably, a scraper adjacent to the cylindrical outer surface of the at least one compression roller is provided.

Preferably, said scraper is configured to scrape the cylindrical outer surface of the at least one compression roller and remove contaminants from said cylindrical outer surface.

The Applicant has verified that, by adopting a back sheet web and a top sheet web composed at least in part of biodegradable fibres, for example cellulose, and by impregnating such webs with a water-based biodegradable glue, these tend to release fibres mixed with glue that could contaminate the slick surface of the compression roller, creating irregularities on it.

The Applicant has found that, by providing a scraper on the slick cylindrical outer surface, the contaminants can be continuously removed during operation of the apparatus.

Preferably, a first compression roller is provided.

Preferably, said first compression roller is mounted at a first distance from said support surface.

Preferably, said first distance is adjustable.

Preferably, said first distance is comprised between 1.2 mm and 2.5 mm, preferably between 1.5 mm and 2 mm, even more preferably between 1.6 mm and 1.9 mm, for example equal to about 1.7 mm.

Preferably, a second compression roller is provided.

Preferably, said second compression roller is configured to compress said multilayer web on said support surface.

Preferably, said second compression roller comprises a slick cylindrical outer surface.

Preferably, said slick cylindrical outer surface corresponds to the entire cylindrical lateral surface of the second compression roller.

Preferably, said second compression roller is arranged downstream of said first compression roller.

The Applicant has found that providing two successive compression rollers allows the multilayer web to be gradually compressed and improves the adherence between the layers of the multilayer web, reducing the penetration of glue into the absorbent core.

Preferably, said second compression roller is mounted at a second distance from said support surface.

Preferably, the second distance is less than the first distance.

Preferably, said second distance is comprised between 1 mm and 2 mm, preferably between 1.2 mm and 1.7 mm, even more preferably between 1.3 mm and 1.6 mm, for example equal to about 1.5 mm.

The Applicant has found that providing a compression level of the second roller that is slightly higher than that of the first roller improves the adhesion between the layers of the multilayer web, while reducing the leakages of glue in the absorbent core.

Preferably, said second distance is adjustable.

Preferably, a scraper adjacent to the cylindrical outer surface of the second compression roller is provided.

Preferably, said scraper of the second compression roller is configured to scrape the cylindrical outer surface of the second compression roller to remove contaminants from said cylindrical outer surface.

Preferably, a supporting drum is provided.

The supporting drum supports and advances the multilayer web coming from the forming unit.

Preferably, said support surface is defined on a cylindrical outer surface of said supporting drum.

Preferably, the at least one compression roller is substantially adjacent to the supporting drum.

Preferably, the rotation axes of the compression roller and of the supporting drum are parallel.

Preferably, the rotation axes of the compression roller and of the supporting drum lie on a plane that is radial both for the compression roller and the supporting drum.

Preferably, said support surface has a width equal to or greater than a width of the multilayer web. The width of the support surface is measured in a direction parallel to the rotation axis of the supporting drum. The width of the multilayer web is measured in a direction orthogonal to a main longitudinal development direction of the multilayer web and parallel to the rotation axis of the supporting drum.

Preferably, the multilayer web is advanced on the support surface.

Preferably, the multilayer web is advanced between the support surface and the at least one compression roller.

Preferably, the multilayer web is advanced between the support surface and the second compression roller.

Preferably, a contact surface configured to receive said multilayer web downstream of said support surface is provided.

Preferably, said contact surface is slick.

Preferably, said contact surface is defined on a contact roller.

Preferably, said apparatus is configured to feed said multilayer web coming from the support surface onto said contact surface.

Preferably, a sealing roller adjacent to said contact surface is provided.

Preferably, said sealing roller is opposite to said counter roller.

Preferably, the sealing roller is configured to seal the top sheet web and the back sheet web around each absorbent core of the multilayer web of the absorbent sanitary article.

Preferably, the sealing roller comprises a cylindrical bottom surface defined at a first radial distance from a rotation axis of said sealing roller.

Preferably, the sealing roller comprises a raised surface defined at a second radial distance from said rotation axis which is greater than the first radial distance.

Preferably, said raised surface is slick.

Preferably, said sealing roller is pressed against said contact surface.

Preferably, said multilayer web is passed between said sealing roller and said contact surface.

Preferably, the distance between the raised surface and the contact surface is less than 1 mm, preferably less than 0.4 mm, even more preferably less than 0.1 mm.

Preferably, the distance between the bottom surface and the contact surface is greater than 1 mm, preferably greater than 1.5 mm, even more preferably greater than 2 mm.

Preferably, said raised surface extends along a perimeter of an absorbent sanitary article being produced.

Preferably, the absorbent core of the absorbent sanitary article being produced is positioned within the perimeter.

Preferably, the sealing roller seals the top sheet web and the back sheet web along the perimeter and around each absorbent core.

Preferably, said bottom surface comprises a first region that externally delimits said raised surface and a second region that is externally delimited by said raised surface.

The part of the multilayer web comprising the absorbent core of the absorbent sanitary article is placed in the inner region.

Preferably, the second region is surrounded by the raised surface.

Preferably, the raised surface is surrounded by the first region.

Preferably, the raised region separates the first region from the second region.

Preferably, the contact roller and the sealing roller have substantially equal dimensions.

Preferably, the rotation axes of the contact roller and of the sealing roller are parallel and lie on a plane that is radial for both the contact roller and the sealing roller.

Preferably, a scraper adjacent to said sealing roller is provided.

In this way, a continuous cleaning of the sealing roller is carried out during processing, ensuring a uniform sealing of the multilayer web and considerably limiting the need for machine downtime. The scraper is adjacent to the raised surface of the sealing roller in order to allow it to be cleaned without being damaged it.

Preferably, said scraper is in contact with the raised surface.

Preferably, a scraper adjacent to said contact roller is provided. In this way, a continuous cleaning of the contact roller is carried out during processing, ensuring a uniform sealing of the multilayer web and considerably limiting the need for machine downtime. The scraper is adjacent to the contact surface of the contact roller in order to guarantee it is cleaned without being damaged.

Preferably, said scraper is in contact with the contact surface.

Preferably, said top sheet web and/or said back sheet web and/or said absorbent core are made of biodegradable material. In this way, the multilayer web of the absorbent sanitary article is, at least partially, biodegradable.

Preferably, said glue is biodegradable glue. In this way, a biodegradable absorbent sanitary article can be produced.

Further characteristics and advantages of the present invention will become clearer from the following detailed description of a preferred embodiment thereof, with reference to the appended drawings and provided by way of indicative and non-limiting example, in which:
- figure 1 is a schematic view of a part of an apparatus for producing an absorbent sanitary article according to the present invention;
- figure 1A is a different schematic view of the apparatus of figure 1;
- figure 2 is a schematic perspective view of a further part of the apparatus according to the present invention;
- figure 3 is a schematic front view of the further part of figure 2 of the apparatus according to the present invention
- figure 4A is an exploded perspective view of an absorbent sanitary article produced with the apparatus of the present invention;
- figure 4B is a schematic sectional view of the absorbent sanitary article of figure 4A.

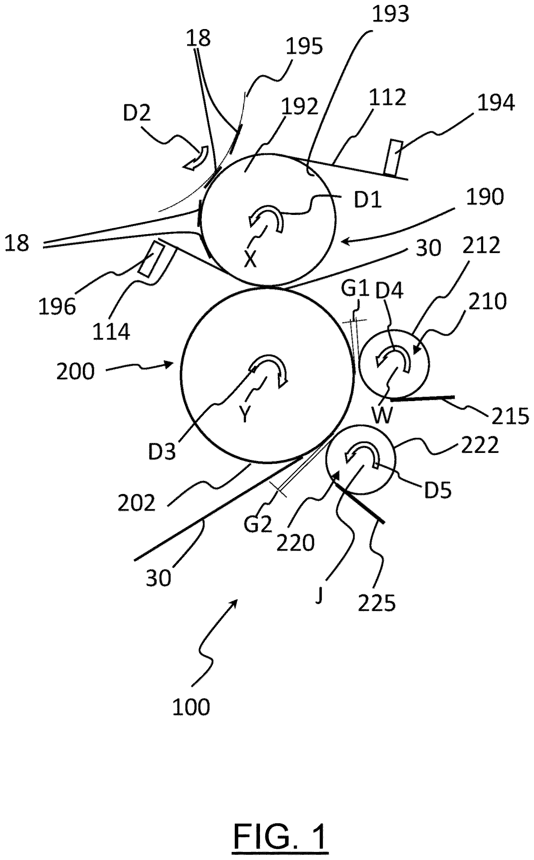

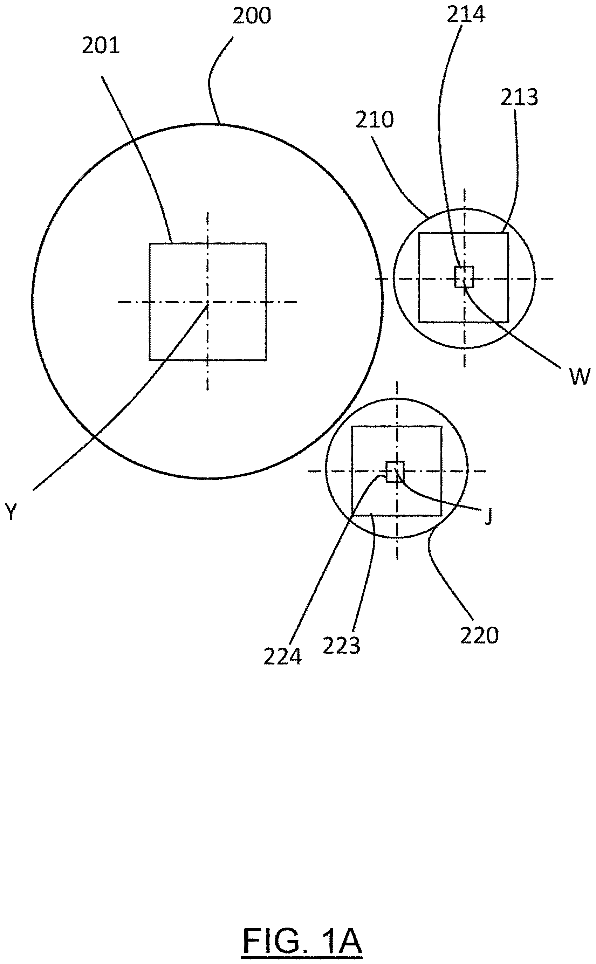

In figures 1-3, reference numeral 100 is used to indicate an apparatus for producing an absorbent sanitary article in accordance with the present invention.

Non-limiting examples of absorbent sanitary articles made with the apparatus 100 are female sanitary napkins, baby diapers, diapers and pads for people suffering from incontinence and the like.

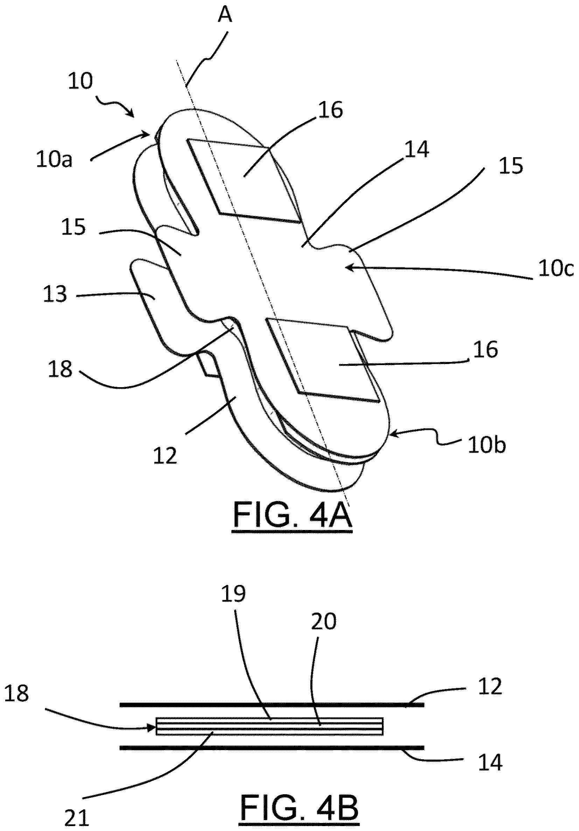

Without thereby losing in generality, explicit reference will be made below to a specific example of an absorbent sanitary article, such as the one illustrated in figures 4A and 4B and indicated with reference numeral 10. This is particularly a female sanitary napkin. When described, it could still find similar application to different types of absorbent sanitary articles.

The article 10 has, along a longitudinal axis A thereof, a front part 10a, a rear part 10b, and a central part 10c arranged between the front part 10a and the rear part 10b.

The article 10 comprises a top sheet 12 and a back sheet 14. Both the top sheet 12 and the back sheet 14 have a substantially rectangular shape, with the two opposite lower sides that are rounded in a convex manner and the two opposite larger sides that are rounded in a concave manner. At the centre of each of the two opposite larger sides of the top sheet 12 and of the back sheet 14 there is a fixing flap 13, 15 which protrudes cantilevered from the larger side, transversely to the longitudinal axis A.

The top sheet 12 and the back sheet 14 define, respectively, an inner face and an outer face of the article 10.

The article 10 can be applied, by means of first adhesive portions 16 placed on the back sheet 14, inside an undergarment, by folding and turning down the two opposite flaps 13, 15 on the outside of the undergarment and by fixing them to it with respective second adhesive portions (not illustrated).

The top sheet 12 is superimposed on the back sheet 14 and is configured to be arranged facing the wearer's body when the article 10 is applied to the undergarment.

The top sheet 12 is made of a material permeable to body fluids. The back sheet 14 comprises a layer substantially impermeable to body fluids. In particular, the top sheet 12 and the back sheet 14 are made of biodegradable materials, preferably cellulose fibres. A liquid containment substrate (not illustrated) made of a water-soluble material, for example, polyvinyl alcohol, not illustrated, is applied to the back sheet.

An absorbent core 18 is arranged between the top sheet 12 and the back sheet 14. This absorbent core 18 has the function of absorbing the body fluids that pass through the top sheet 12 and retaining them with no leaks even for several hours. The absorbent core 18 is made of absorbent material, comprising a matrix of fibres, for example of cellulose, and an absorbent powder (SAP) dispersed in the matrix of fibres, for example based on carboxymethylcellulose (CMC). In this way, the absorbent core 18 is biodegradable. The absorbent core 18 extends longitudinally from the front part 10a to the rear part 10b and has a substantially oval shape, tight at the central portion 10c.

Figures 1-3 depict parts of the apparatus 100 that allows producing the absorbent sanitary article 10.

The apparatus 100 comprises a forming unit 190 configured to form a multilayer web 30 of the absorbent sanitary article 10. The multilayer web 30 comprises a top sheet web 112, a back sheet web 114 and a plurality of absorbent cores 18 placed in succession between the top sheet web 112 and the back sheet web 114. In successive processing, the multilayer web 30 will be cut into discrete elements each corresponding to a single absorbent article 10 so that the top sheet 12 of the absorbent article 10 will be obtained from the top sheet web 112 and the back sheet 14 of the absorbent article 10, with one of the absorbent cores 18 placed therebetween will be obtained from the back sheet web 114.

In the non-limiting example of figure 1, the forming unit 190 comprises a forming drum 192 rotatable about the rotation axis X. The forming drum 192 comprises an outer surface 193, preferably aspirated.

The forming drum 192 is configured to rotate in the direction D1 (which is a counterclockwise direction in figure 1) so as to advance the outer surface 193 along a forming path.

The forming unit 190 is configured to feed the top sheet web 112 onto the forming drum 192, in particular directly onto the outer surface 193.

A first glue dispenser 194 is configured to dispense a liquid, preferably water-based, glue onto the top sheet web 112 before the top sheet web 112 is fed on the forming drum 192, so as to impregnate it with glue.

In the non-limiting example of figure 1, the forming unit 190 comprises a transport drum 195 (only partially shown) for transporting absorbent cores 18. The transport drum 195 is configured to retain a plurality of absorbent cores 18, preferably by suction, and to advance them in a direction D2 (which is a clockwise direction in figure 1), opposite to the direction D1 of the forming drum, by rotating around its rotation axis (not illustrated).

The transport drum 195 is substantially tangent to the forming drum 192 and is configured to deposit the absorbent cores 18 on the forming drum 192 superimposing them on the top sheet web 112.

The forming unit 190 is configured to feed the back sheet web 114 onto the forming drum 192, superimposing it on the top sheet web 112 and the absorbent cores 18 superimposed thereon. In this way the absorbent cores 18 remain placed between the top sheet web 112 and the back sheet web 114 and the multilayer web 30 is formed.

A second glue dispenser 196 is configured to dispense a liquid, preferably water-based, glue onto the back sheet web 114 before it is fed onto the forming drum 192, so as to impregnate it with glue.

In an alternative non-illustrated embodiment of the present invention, only one between the first glue dispenser 194 and the second glue dispenser 196 could be provided so as to impregnate only one between the top sheet web 112 and the back sheet web 114.

A supporting drum 200 is adjacent to the forming drum 192, substantially tangent to it. The supporting drum 200 is rotatably mounted on a frame 201, illustrated in figure 1A. The supporting drum 200 comprises a support surface 202. The support surface 202 is defined on a cylindrical outer surface of the supporting drum 200. The support surface 202 is preferably slick and has no clear knurls, holes, or other surface irregularities.

The supporting drum 200 is rotatable about a rotation axis Y. The rotation axis Y is parallel to the rotation axis X. The supporting drum 200 is configured to rotate in the direction D3 (which is a clockwise direction in figure 1), opposite to the direction D2.

In the non-limiting example illustrated in figure 1, the forming drum 192 is placed above the supporting drum 200. The diameter of the forming drum 192 is less than the diameter of the supporting drum 200.

The forming drum 192 is configured to feed the multilayer web 30 onto the supporting drum 200. The multilayer web 30 is advanced in the direction D1 on the forming surface 193 of the forming drum 192 and passed between the forming drum 192 and the supporting drum 200. When the multilayer web 30 arrives between the forming drum 192 and the supporting drum 200, the multilayer web 30 is dropped by the forming drum 192, for example by ceasing the suction action towards the forming surface 193, and taken over by the supporting drum 200.

The multilayer web 30 is advanced on the support surface 202 in the direction D3.

The apparatus 100 comprises a first compression roller 210 adjacent to the supporting drum 200 and configured to press the multilayer web 30 against the support surface 202. The first compression roller 210 has a slick cylindrical outer surface 212, thus free of knurls, holes, or other obvious surface irregularities. Preferably, the cylindrical outer surface 212 corresponds to the entire lateral surface of the first compression roller 210.

The first compression roller 210 is rotatably mounted on a first supporting body 213 (illustrated in figure 1A), preferably in an idle manner. The first compression roller 210 is rotatable about a first rotation axis W parallel to the rotation axis Y of the supporting drum 200. The first compression roller 210 is configured to rotate about the first rotation axis W in a direction D4 (which is a counterclockwise rotation in figure 1) opposite to the direction D3 of the supporting drum 200.

Preferably, the first supporting body 213 is connected to first adjustment members 214 (illustrated in figure 1A) which are configured to allow an adjustment of the position of the first compression roller 210 with respect to the supporting drum 200. The first adjustment members 214 may for example comprise threaded members such as bolts and nuts. The first adjustment members 214 are configured to allow the position of the first compression roller 210 to be adjusted in a direction orthogonal to the support surface 202. This direction is radial with respect to the rotation axis Y of the supporting drum 200.

The apparatus 100 comprises a scraper 215 configured to scrape the cylindrical outer surface 212 to remove glue residues and/or fibres. The scraper 215 is mounted on the first supporting body 213 and has a free edge in sliding contact with the cylindrical outer surface 212.

The first compression roller 210 is positioned at a first distance G1 from the support surface 202. The first distance G1 defines a gap between the supporting drum 200 and the first compression roller 210 through which the multilayer web 30 is passed. The first distance G1 is adjustable by means of the first adjustment members 214. In the preferred embodiment, the first distance G1 is equal to approximately 1.7 mm.

The multilayer web 30 is advanced on the support surface 202, in particular by rotation of the supporting drum 200, and passed between the support surface 202 and the first compression roller 210, so as to be compressed between the two. The compression of the multilayer web 30 improves the adherence between the individual glue-impregnated layers of the multilayer web 30.

The apparatus 100 comprises a second compression roller 220 adjacent to the supporting drum 200 and arranged downstream of the first compression roller 210 with respect to the rotation direction D3 of the supporting drum 200. The second compression roller 220 is configured to press the multilayer web 30 against the support surface 202. The second compression roller 220 has a slick cylindrical outer surface 222, thus free of knurls, holes, or other obvious surface irregularities. Preferably, the cylindrical outer surface 222 corresponds to the entire lateral surface of the first compression roller 210. In the non-limiting example illustrated in figure 1, the first and second compression roller 210 and 220 have substantially the same diameter.

The second compression roller 220 is rotatably mounted on a second supporting body 223 (illustrated in figure 1A), preferably in an idle manner. The second compression roller 220 is rotatable about a second rotation axis J parallel to the rotation axis Y of the supporting drum 200. The second compression roller 220 is configured to rotate about the second rotation axis J in a direction D5 (which is a counterclockwise direction in figure 1) opposite to the direction D3 of the supporting drum 200.

Preferably, the second supporting body 223 is connected to second adjustment members 224 configured to allow a position adjustment of the second compression roller 220 with respect to the supporting drum 200. The second adjustment members 224 may for example comprise threaded members such as bolts and nuts. The second adjustment members 224 are configured to allow the position of the second compression roller 220 to be adjusted in a direction orthogonal to the support surface 202. This direction is radial with respect to the rotation axis Y of the supporting drum 200.

The apparatus 100 comprises a scraper 225 configured to scrape the cylindrical outer surface 222 to remove glue residues and/or fibres. The scraper 225 is mounted on the second supporting body 223 and has a free edge in sliding contact with the cylindrical outer surface 222.

The second compression roller 220 is positioned at a second distance G2 from the support surface 202. The second distance G2 defines a gap between the supporting drum 200 and the second compression roller 220 through which the multilayer web 30 is passed. The second distance G2 is adjustable by the second adjustment members 224. The second distance G2 is less than the first distance G1. In the preferred embodiment, the second distance G2 is equal to approximately 1.5 mm.

The multilayer web 30 advancing on the support surface 202 is passed firstly between the support surface 202 and the first compression roller 210 and then between the support surface 202 and the second compression roller 220 so as to be gradually compressed and improve the adherence between the individual glue-impregnated layers of the multilayer web 30 reducing glue leakages.

In an alternative non-illustrated embodiment of the present invention, a single compression roller could be provided instead of the two compression rollers 210 and 220.

The apparatus 100 is configured to allow the multilayer web 30 to leave the support surface 202 downstream of the second compression roller 220 and to be moved along an advancement path to be subjected to subsequent processing.

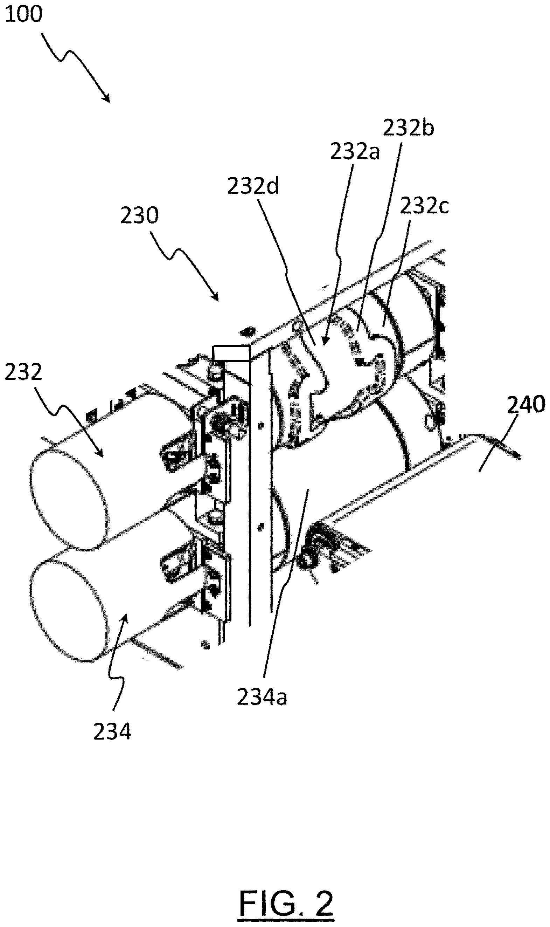

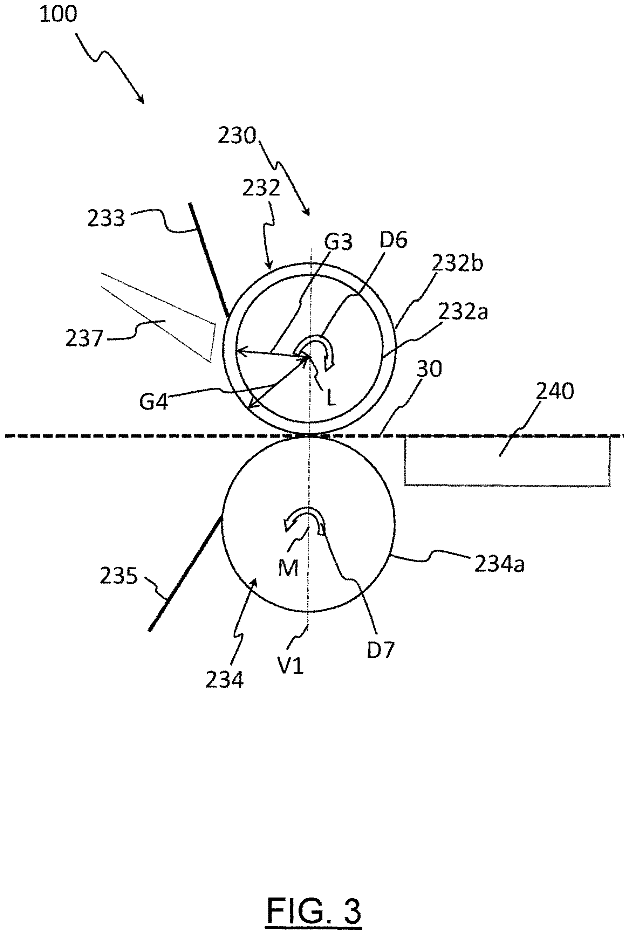

The apparatus 100 comprises, downstream of the supporting drum 200, a sealing station 230, shown in figures 2 and 3. Further processing stations may possibly be placed between the supporting drum 200 and the sealing station 230 along the advancement path of the multilayer web 30.

The sealing station 230 seals the top sheet web 112 and the back sheet web 114 around each absorbent core 18 of the multilayer web 30.

In the non-limiting example of figures 2 and 3, the multilayer web 30 is fed to the sealing station by a conveyor belt 240. The conveyor belt 240 may for example be configured to receive the multilayer web 30 from the supporting drum 200.

The sealing station 230 comprises a sealing roller 232 opposite to a contact roller 234.

The sealing roller 232 rotates about the rotation axis L in the direction D6 (which is a clockwise direction in figure 3).

The contact roller 234 rotates about the rotation axis M, parallel to the rotation axis L, in the direction D7 (which is a counterclockwise direction in figure 3), opposite to the direction D6 of the sealing roller 232.

In the non-limiting example illustrated in figure 3, the rotation axes L and M lie on a trace plane V1 which is substantially vertical.

The contact roller 234 is placed substantially tangent to the sealing roller 232, except for the thickness of the portion of multilayer web 30 to be sealed. The rotation axes L and M lie on a plane that is radial for both the contact roller 234 and the sealing roller 232.

In the non-limiting example illustrated in figure 3, the sealing roller 232 and the contact roller 234 have substantially the same outer diameter.

A slick contact surface 234a is defined on a cylindrical outer surface of the contact roller 234.

The sealing roller 232 comprises a cylindrical bottom surface 232a defined at a first radial distance G3 from the rotation axis L and a slick raised surface 232b, defined at a second radial distance G4 from the rotation axis L which is greater than the first radial distance G3 (figure 3).

The sealing roller 232 is pressed against the contact roller 234 so that the slick raised surface 232b is pressed against the slick contact surface 234a.

Referring to figure 2, the raised surface 232b extends along the perimeter of the absorbent sanitary article being produced such that, when the multilayer web 30 passes between the sealing roller 232 and the sealing roller 234, the raised surface 232b presses on a region in the multilayer web 30 comprising the top sheet web 112 and the back sheet web 114 superimposed and not comprising any absorbent core 18.

The bottom surface 232a comprises a first region 232c external to the raised surface 232b. The first region 232c extends outside the perimeter along which the raised surface 232b extends.

The bottom surface 232a comprises a second region 232d delimited by the raised surface 232b. The part of the multilayer web 30 comprising the absorbent core 18 of the absorbent sanitary article 10 to be produced is operatively positioned in the inner region 232d such that this part is not pressed by the raised surface 232b against the contact surface 234a.

The raised surface 232b is configured to seal the top sheet web 112 and the back sheet web 114 around the absorbent cores 18 by pressing the top sheet web 112 and the back sheet web 114 against the contact surface 234a.

The apparatus 100 comprises a scraper 235 adjacent to the contact surface 234a so as to clean it without damaging it.

The apparatus 100 further comprises a scraper 233 adjacent to the raised surface 232b so as to clean it without damaging it. In addition, near the scraper 233 and adjacent to the raised surface 232b, a suction duct 237 is provided, configured to suck excess glue and processing residues.

Obviously, a person skilled in the art, in order to satisfy specific and contingent needs, can make numerous modifications and variations to the invention described above while remaining within the scope of protection defined by the following claims.

## Claims

1. Apparatus (100) for producing an absorbent sanitary article (10), comprising:
- a slick support surface (202);
- a forming unit (190) configured to form a multilayer web (30) of an absorbent sanitary article (10), the multilayer web (30) comprising a top sheet web (112), a back sheet web (114) and a plurality of absorbent cores (18) placed between said top sheet web (112) and said back sheet web (114), and to feed said multilayer web (30) on said support surface (202);
- at least one glue dispenser (194; 196) configured to dispense a liquid glue onto said back sheet web (114) and/or onto said top sheet web (112);
- at least one compression roller (210; 220) adjacent to said support surface (202) and configured to compress said multilayer web (30) on said support surface (202), said at least one compression roller (210; 220) comprising a slick cylindrical outer surface (212; 222).

2. Apparatus (100) according to claim 1, wherein said at least one compression roller (210; 220) is mounted at a respective adjustable distance (G1; G2) from said support surface (202).

3. Apparatus (100) according to claim 1 or 2, comprising a scraper (215; 225) adjacent to the cylindrical outer surface (212, 222) of the at least one compression roller (210, 220).

4. Apparatus (100) according to any one of the previous claims, wherein said at least one compression roller (210; 220) comprises a first compression roller (210) and a second compression roller (220), wherein said second compression roller (220) is adj acent to said support surface (202) and arranged downstream of said first compression roller (210).

5. Apparatus (100) according to claim 4, wherein said first compression roller (210) is mounted at a first distance (G1) from said support surface (202) and said second compression roller (220) is mounted at a second distance (G2) from said support surface (202) which is less than said first distance (G1).

6. Apparatus (100) according to any one of the previous claims, comprising a supporting drum (200), said support surface (202) being defined on a cylindrical outer surface of said supporting drum (200).

7. Apparatus (100) according to any one of the previous claims, comprising:
- a slick contact surface (234a) configured to receive said multilayer web (30) downstream of said support surface (202);
- a sealing roller (232) adjacent to said contact surface (234a) and comprising a cylindrical bottom surface (232a) defined at a first radial distance (G3) from a rotation axis (L) of said sealing roller (232) and a slick raised surface (232b) defined at a second radial distance (G4) from said rotation axis (L) which is greater than the first radial distance (G3);
- wherein said sealing roller (232) is pressed against said contact surface (234a).

8. Apparatus (100) according to claim 7, wherein said raised surface (232b) extends along a perimeter of the absorbent sanitary article (10) being produced, and wherein said bottom surface (232a) comprises a first region (232c) that externally delimits said raised surface (232b) and a second region (232d) that is externally delimited by said raised surface (232b).

9. Method for producing an absorbent sanitary article (10), comprising:
- forming a multilayer web (30) of an absorbent sanitary article (10) comprising a top sheet web (112), a back sheet web (114) and a plurality of absorbent cores (18) placed between said top sheet web (112) and said back sheet web (114);
- feeding said multilayer web (30) onto a slick support surface (202);
- dispensing liquid biodegradable glue on said back sheet web (114) and/or on said top sheet web (112);
- compressing said multilayer web (30) between at least one compression roller (210; 220) comprising a slick cylindrical outer surface (212; 222) and said support surface (202).
